**Europäisches Patentamt**

**European Patent Office**

**Office européen des brevets**

(19)

(11) Veröffentlichungsnummer: **0 333 730 B1**

(12)

# EUROPÄISCHE PATENTSCHRIFT

(45) Veröffentlichungstag der Patentschrift: **21.10.92**

(51) Int. Cl.5: **A61L 33/00**, C08B 37/08

(21) Anmeldenummer: **87907576.0**

(22) Anmeldetag: **20.11.87**

(86) Internationale Anmeldenummer:
**PCT/DE87/00535**

(87) Internationale Veröffentlichungsnummer:
**WO 88/03813 (02.06.88 88/12)**

(54) **VERFAHREN ZUR HERSTELLUNG VON NICHT-THROMBOGENEN SUBSTRATEN.**

(30) Priorität: **20.11.86 DE 3639561**

(43) Veröffentlichungstag der Anmeldung:
**27.09.89 Patentblatt 89/39**

(45) Bekanntmachung des Hinweises auf die
Patenterteilung:
**21.10.92 Patentblatt 92/43**

(84) Benannte Vertragsstaaten:
**AT BE CH FR GB IT LI LU NL SE**

(56) Entgegenhaltungen:
**EP-A- 0 086 186**
**EP-A- 0 199 033**
**WO-A-83/03536**
**GB-A- 2 041 377**

**Journal of Biological Chemistry, vol. 256, Nr. 15, 10 Austust 1981, (US), B.G.J. Salisbury et al.: "Isolation and preliminary characterisation of proteoglycans dissociatively extracted from human aorta" pages 8050-8057 see page 8050**

(73) Patentinhaber: **KELLER, Ruprecht**
**Buchenweg 2**
**W-5100 Aachen(DE)**

Patentinhaber: **BAUMANN, Hanno**
**Augustinerweg 23**
**W-5100 Aachen(DE)**

(72) Erfinder: **KELLER, Ruprecht**
**Buchenweg 2**
**W-5100 Aachen(DE)**
Erfinder: **BAUMANN, Hanno**
**Augustinerweg 23**
**W-5100 Aachen(DE)**

(74) Vertreter: **Dr. Fuchs, Dr. Luderschmidt**
**Dipl.-Phys. Seids, Dr. Mehler Patentanwälte**
**Abraham-Lincoln-Strasse 7**
**W-6200 Wiesbaden(DE)**

Americal Journal of Surgery, vol. 150, August 1985, K.S. Baker et al.: "Endothelialization of human collagen surfaces with human adult entothelial cells", pages 197-200 see page 200

**Beschreibung**

Die vorliegende Erfindung betrifft ein Verfahren zur Herstellung von hämokompatiblen Substraten durch Einarbeitung, Adhäsion und/oder Modifizierung und Verankerung von nicht-thrombogenem Endothelzelloberflächen-Polysaccharid (HS I) in seiner Peptid-gebundenen oder freien Form in und/oder auf synthetische und Bio-Polymere (Substrate) mittels physikalischer Verteilung, Adhäsion an der Oberfläche und/oder chemischer Verankerung, die als blutverträgliche Substrate in der Medizin eingesetzt werden können. Diese Polymere können in Form von Fasern, Hohlfasern, Membranen, Organersatzteilen, Kanülen, Spritzen, Schläuchen, Blutbehältern oder in anderer Form vorliegen oder dazu verarbeitet werden.

Es ist bekannt, daß unphysiologische Polymere ohne zusätzliche Hilfsmaßnahmen bei Blutkontakt mehr oder weniger rasch zur Blutgerinnung führen. Weiterhin ist bekannt, daß die Strömungsverhältnisse des Blutes, die u.a. durch die Gestalt, Oberflächenbeschaffenheit und elastischen Eigenschaften von Substraten, beeinflußt werden, den Gerinnungsprozeß des Blutes ebenfalls stark beeinflussen. Wenn jedoch geeignete Polymere, wie z.B. EP-Copolymere, Cellulose, fluoriertes Polyethylen, Polyetherurethan, Polyethercarbonat, Hydrogele von z.B. Polyacrylamid ausgewählt werden, nimmt die Blutgerinnung nach Polymerkontakt deutlich ab.

Eine andere Möglichkeit besteht darin, die Blutgerinnung durch Überziehen von hydrophoben Polymeren mit Albumin weiter herabzusetzen. Eine weitere Möglichkeit zur Herabsetzung der Koagulation des Blutes ist der Einsatz von sulfonierten und sulfatierten Produkten, wie z.B. sulfatiertes Polystyrol oder vinylsulfonsäurehaltige Copolymere.

Die größten blutgerinnungshemmenden Effekte wurden durch Einbau oder Oberflächen-Modifizierung von Polymeren mit blutgerinnungshemmenden Mitteln, wie Heparin oder Heparinoide, erzielt. Schon der Einschluß von Heparin in mikroporöses Kunststoffmaterial, aus dem es leicht herausdiffundieren kann, führt zu einer antikoagulierenden Wirkung. Deutlichere Effekte wurden durch oberflächliche Modifizierung von z.B. Styrol mit anschließender salzartiger Bindung von Heparin erzielt.

Durch die bisher produzierten Materialien ist es aber noch nicht gelungen, eine Blutverträglichkeit zu erreichen, wie sie z.B. die intakte Endothelzelloberfläche auf der luminalen Seite des Blutgefäßes bewirkt.

Aufgabe der vorliegenden Erfindung ist ein Verfahren zur Herstellung von hämokompatiblen, polymeren Substraten.

Gegenstand der vorliegenden Erfindung ist ein Verfahren zur Einlagerung, Adsorption oder Bindung eines biologisch und speziell gerinnungsphysiologisch inerten Endothelzelloberflächen-Polysaccharids in seiner freien oder peptidgebundenen Form an oder in geeignete Polymere. Diese Einlagerung, Adsorption oder Bindung kann über van der Waals-Wechselwirkungen, Salzbrücken, Chelatbindungen oder durch kovalente Bindung erreicht werden. Die Blutverträglichkeit der so gewonnenen modifizierten Polymeren ist gegenüber allen bisher bekannten Polymeren deutlich verbessert.

Die so modifizierten Polymeren sollen Einsatz finden in Form von künstlichen Organen, Membranen, Hohlfasern, Oxigenatoren, Spritzen, Schläuchen, Behältern, faserhaltigen Gebilden, die dadurch gekennzeichnet sind, daß

(1) HSI eingearbeitet oder immobilisiert oder auf der Oberfläche verankert wird in synthetische und Biopolymere nach den bekannten Methoden der Immobilisierung von Enzymen, Membranherstellung, der Kunststoffverarbeitung und den Methoden der Polymerchemie über van der Waals-Kräfte, hydrophobe Interaktionen, Chelatbindungen und salzartige Bindungen,

(2) HSI kovalent verankert wird an synthetischen Polymeren und Biopolymeren nach den Methoden der Peptidchemie, Proteinchemie, Zuckerchemie oder Polymerchemie.

Unter dem Begriff HSI wird ein Endothelzelloberflächen-Proteo-Polysaccharid mit einem Molekulargewicht von etwa 195 000 und einem Molekulargewicht des Polysaccharidteils von 35 000 bei 3 - 4 Polysaccharidketten/Molekül, und einem Peptidanteil von 55 000 verstanden. Der Polysaccharidanteil, eine Untereinheit des Moleküls, die über Proteolyse oder Alkaliabbau zugänglich ist, hat sich in allen biologischen Aggregations- und Funktionstesten als völlig inert erwiesen, z.B. in Bezug auf

- Wechselwirkung mit den Faktoren der Blutgerinnung,
- Wechselwirkung mit Antithrombin III,
- Wechselwirkung mit Heparin-bindenden Wachstumsfaktoren, wie z.B. Platelet derived growth factor etc.,
- Wechselwirkung mit Bindegewebsstrukturpolymeren, wie Kollagen I - VI, Laminin, Fibronectin, Nidogen und Entactin,
- Wechselwirkung mit Thrombozyten.

Diese Substanz kann als Polysaccharidanteil mit oder ohne verbleibendem Peptidrest für die Bindung an Polymere zur Herstellung hämokompatibler Substrate eingesetzt werden. Die Langzeit-Blutverträglichkeit von Peptid-gebundenem HS I-Polysaccharid kann durch Bindung von Plasmaproteinen an den verbleibenden Peptidrest oder Immunisierung bei Verwendung von HS I aus anderen Spezies eingeschränkt sein. In einem solchen Fall wird der Peptidrest durch weitere Proteolyse verkürzt oder, falls erforderlich, durch Einwirkung von 0,5 M NaOH restlos entfernt.

HS I wird aus dem konditionierten Medium von massenkultivierten Endothelzellen oder aus den Endothelzellen selbst nach Molgewicht, Hydrophobizität und Ladung gereinigt:
Bovine aortale Endothelzellen, oder Endothelzellen aus anderen Spezies und anderen Blutgefäßen, bzw. Endothelzellen aus anderen Geweben, wie z.B. Cornea, Liquorhöhlen etc., werden durch Behandlung mit Kollagenase, durch mechanische Ablösung, durch Behandlung mit anderen lytischen Reagenzien, wie z.B. Heparinase und/oder Heparitinase, durch Behandlung mit Trypsin oder anderen Proteasen, durch Einwirkung von EDTA, EGTA oder anderen Chelatbildnern aus dem Gewebe gelöst und in herkömmlichen Gewebekulturflaschen, in Petrischalen, in Hollow-Fiber-Perfusionskulturen, auf Beads, in Rollerflaschen oder in anderer Weise in großer Anzahl gezüchtet ($2 \times 10^9$ Zellen/ Präparation). Die Aufzüchtung erfolgt nach den herkömmlichen Techniken der Gewebe- und Zellkultur.

Die Zellen werden, wie oben beschrieben, von der Unterlage gelöst, durch wiederholtes Einfrieren und Auftauen, durch Ultraschallbehandlung, durch Behandlung in einem Potter oder durch andere Weise homogenisiert, bei 10 000 x g abzentrifugiert und der Überstand mit Detergenzien, vorzugsweise 2 % SDS, gelöst.

Als eine weitere Quelle für HS I kann das konditionierte Medium von Endothelzellen dienen. Hierzu wird das Medium zunächst mit Chondroitinase ABC inkubiert, in Gegenwart von Chelatbildnern, wie z.B. EDTA, im Rotationsverdampfer eingeengt und dann prinzipiell in der gleichen Weise, wie für das Material zellulären Ursprungs beschrieben, aufgearbeitet.

Die HS I-haltigen Materialien werden an Sepharose CL-6B oder Sepharose CL-4B chromatographiert. Als Säulendimensionen werden Durchmesser zu Höhen-Verhältnisse von 1 : 10 - 1 : 50 verwendet (z.B. 10 x 200 cm). Als Elutionsmittel kann 0,13 M Tris/HCl, 0,1 % SDS pH 7,4 oder ein anderes geeignetes Laufmittel Verwendung finden. Der Nachweis des HS I in dem Präparationsschritt kann durch Reaktion von Aliquots aus den Chromatographiefraktionen in Nachweisreaktionen für Zukker, wie z.B. die Orcinreaktion, in Nachweisreaktionen für Uronsäuren, wie z.B. die Carbazolreaktion, durch Zugabe einer geringen Menge von radioaktiv markiertem HS I vor der Sepharose-Chromatographie, oder auf andere Weise erfolgen.

Die so identifizierten HS I-haltigen Chromatographiefraktionen werden im Rotationsverdampfer 1 : 10 eingeengt, durch Chromatographie an Sephadex G-25, oder durch Dialyse, durch Ultrafiltration an Ultrafiltrationsmembranen, durch Ausfällen in 80 % Ethanol oder auf andere Weise entsalzt und danach in 3 - 7 M Harnstoff, Detergenzien, wie z.B. 0,1 % Triton X-100 und Puffersalzen, wie z.B. 0,1 M NaAc pH 6,5, aufgenommen. Die Lösung wird auf eine Anionenaustauschersäule, wie z.B. DEAE-Cellulose, in Gegenwart von 7 M Harnstoff, Detergenzien und Puffersalzen aufgetragen und mit einem Salz-Gradienten (z.B. 0 - 1 M NaCl) wieder von der Säule eluiert.

Als weitere Reinigungsschritte stehen die folgenden Techniken zur Verfügung:
- Dichtegradienten-Ultrazentrifugation in einem CsCl-Gradienten bei einer mittleren Dichte von vorzugsweise 1,4 g/ml;
- weitere Zyklen von Gelchromatographie/Ionenaustauscherchromatographie;
- Chromatographie an lipophilen Gelen, wie z.B. Octylsepharose;
- HPLC an Ionenaustauschersäulen, Silicagelsäulen, reversed-phase-Säulen und an Gelsäulen.

Die nachfolgenden Kriterien werden zur Prüfung der Homogenität einer HS I-Präparation herangezogen:
1. die Nicht-Nachweisbarkeit von Galaktosamin in der Bausteinanalyse zeigt die Freiheit von kontaminierendem Chondroitinsulfat an;
2. ein Absorptionsmaximum bei 280 nm und ein Minimum bei 260 nm zeigt die Freiheit von kontaminierender RNS oder DNS an;
3. eine Bande in der NU-Sieve-Agarose Gelelektrophorese und eine Bande (bei 55 000) in der SDS-Gelelektrophorese zeigt die Abwesenheit von kontaminierenden Proteinen an;
4. nach Inkubation in 0,5 M NaOH für 12 h bei 4°C wird ein hochmolekulares Polysaccharid gefunden.

Als Modifikation der Präparation kann auch das HS I-Polysaccharid bzw. -Peptidopolysaccharid durch Alkalidegradation (z. B. Inkubation in 0,5 M NaOH während 12 h bei 4°C) oder proteolytischen Abbau (z.B. mit Papain oder mit Pronase) oder andere Verfahren in jeder Stufe der Präparation, auch aus den unbehandelten Zellen oder aus dem unbehandelten Medium, freigesetzt und über Gelchromatographie, enzymatischen Abbau von kontaminierenden Biopolymeren, Ionenaustauscherchromatographie, Dichtegradientenultrazentrifugation, HPLC an Gelpermeationssäulen, an Silicagelen und/oder an reversed-phase-Säulen gereinigt werden. Mit Ausnahme des vorstehend angeführten Punktes 3. finden alle oben dargestellten Homogenitätskriterien für eine solche Präparation Verwendung.

Unter dem Begriff "synthetische Polymere und Biopolymere" sollen alle bekannten natürlichen Polymeren und bisher synthetisch hergestellten Polymeren, die als Homo- und verschiedene Copolymere mit unterschiedlicher Taktizität, unterschiedlichem Molgewicht, unterschiedlicher Sequenzanordnung der Bausteine u.a. statistischer und/oder alternierender Reihenfolge; Blockcopolymere mit unterschiedlichen Sequenzlängenverteilungen, Triblockcopolymere, Ionomere, Pfropfcopolymere, Polymere mit unterschiedlichem Vernetzungsgrad, sowie Polymere, die durch polymeranaloge Reaktionen modifiziert werden, verstanden werden. Die erfindungsgemäß im folgenden aufgeführten Polymeren sind nur eine kleine Auswahl von Polymeren, die durch weitere Copolymerisationen, Pfropfung oder polymeranaloge Reaktionen zusätzlich modifiziert werden können und damit die erweiterte Auswahl von geeigneten synthetischen und Biopolymeren ergeben. Beispiele der kleineren Auswahl von geeigneten synthetischen und Biopolymeren sind:

Polyolefine, Polyethylen (HDPE, LDPE, LLPE), fluoriertes Ethylen, Copolymere des Ethylens mit Buten-(1), Penten-(1), Hexen-(1), Copolymere des Ethylens und Propylens, EPR-Kautschuk, oder EPT-Kautschuk (dritte Komponente mit Dienstruktur u.a. Dicyclopentadien, Ethylidennorbonen, Methylendomethylenhexahydro-naphthalin, cis,cis-Cyclooctadien-1,5, Hexadien-1,4), Hexyn (1-Hexen-methylhexadien),

Ethylen-Vinylacetat-Copolymere, Ethylen-Methacrylsäure-Copolymere, Ethylen-N-Vinylcarbazol, Ethylen-Trifluorchlorethylen, Polypropylen, Polybuten-(1), Poly-4-(methylpenten-(1)), Polyisobutylen-Copolymere, Isobutylen-Styrol-Copolymere, Butylkautschuk, Polystyrol und modifiziertes Styrol, chlormethyliertes Styrol, sulfoniertes Styrol, Poly-(4-aminostyrol), Styrol-Acrylnitril-Copolymere, Styrol-Acrylnitril-Butadien-Copolymere, Acrylnitril-Styrol-Acrylester-Copolymere, Styrol-Butadien-Copolymere, Styrol-Divinylbenzol-Copolymere, Polydiene in der cis,trans-, in der 1,2- und in der 3,4-Konfiguration, Butadien, Isopren, gereinigter Naturkautschuk, Chloropren, Styrol, Butadien-Copolymere (SBR), Triblockpolymere (SBS), NBR-Acrylnitril-Butadien-Copolymere, Poly-(2,3-dimethylbutadien),ein Triblock-Copolymeres aus Polybutadien, terminiert mit cycloaliphatischen sekundären Aminen oder Benzyl-L-glutamat oder Polypeptiden oder N-Carbobenzoxylysin, Poly-(alkenamere)-Polypentenamere, Poly-(1-hexen-methylhexadien), Polyphenylen, Poly-(p-xylylen), Polyvinylacetat, Vinylacetat-Vinylstearat-Copolymere, Vinylacetat-Vinylpivalat-Copolymere, Vinylacetat-Vinylchlorid-Copolymere, Polyvinylalkohol, Polyvinylformal, Polyvinylbutyral, Polyvinylether, Poly(N-vinylcarbazol), Poly-N-vinylpyrrolidon, Poly-(4-vinylpyridin), Poly-(2-vinylpyridiniumoxid), Poly-(2-methyl-5-vinylpyridin), Butadien-(2-methyl-5-vinylpyridin)-Copolymere, Polytetrafluorethylen, Tetrafluorethylen-Hexafluorpropylen-Copolymere, Tetrafluorethylen-Perfluorpropylvinylether-Copolymere, Tetrafluorethylen-Ethylen-Copolymere, Tetrafluorethylen-Trifluornitrosomethan-Copolymere, Tetrafluorethylen-Perfluormethylvinylether-Copolymere, Tetrafluorethylen-(Perfluor-4-cyanobutylvinylether)-Copolymere, Poly-(trifluorchlorethylen), Trifluorchlorethylen-Ethylen-Copolymere, Polyvinylidenfluorid, Hexafluorisobutylen-Vinylidenfluorid-Copolymere, Polyvinylfluorid, Polyvinylchlorid, schlagfestes PVC durch Beimischen von ABS, MBS, NBR, chloriertem PE, EVAC oder Polyacrylaten, Weich-PVC, nachloriertes PVC, Vinylchlorid-Vinylacetat-Copolymere, Vinylchlorid-Propylen-Copolymere, Polyvinylidenchlorid, Vinylchlorid-Vinylidenchlorid-Copolymere, Vinylidenchlorid-Acrylnitril-Copolymere, Polyacrylsäure, Acrylsäure-Itakonsäure-Copolymere, Acrylsäure-Methacrylsäure-Copolymere, Acrylsäureester-Acrylnitril-Copolymere, Acrylsäureester-2-Chlorethylvinylether-Copolymere, Poly-(1,1-dihydroperfluorbutylacrylat), Poly-(3-perfluormethoxy-1,1-dihydroperfluorpropylacrylat), Polysulfon, Polyacrolein, Polyacrylamid, Acrylsäure-Acrylamid-Copolymere, Acrylamid-Maleinsäure-Copolymere, Acrylamid-Hydroxymethylmethacrylat-Copolymere, Acrylamid-Methylmethacrylat-Copolymere, Acrylamid-Methylacrylat-Copolymere, Acrylamid-Maleinsäureanhydrid-Copolymere, Acrylamid-Methacrylsäureanhydrid-Copolymere, Acrylamid-Anilinoacrylamid-Copolymere, Acrylamid-(N-acrylol-4-carboxymethyl-2,2'-dimethylthiazolin)-Copolymere, Polymethacrylamid, Methacrylsäure-Methacrylnitril-Copolymere, Methacrylsäure-3-Fluorstyrol-Copolymere, Methacrylsäure-4-Fluorstyrol-Copolymere, Methacrylsäure-3-Fluoranilid-Copolymere, nitrierte Copolymere von Methacrylsäure mit Methacrylsäure-3-fluoroanilid oder Fluorostyrol, oder Copolymere von Methacrylsäure mit 3-4-Isothiocyanatostyrol, oder N-Vinylpyrrolidon mit Maleinsäureanhydrid, oder Polyvinylalkohol und Polyallylalkohol, Polyacrylnitril, Acrylnitril-2-Vinylpyridin-Copolymere, Acrylnitril-Methallylsulfonat-Copolymere, Acrylnitril-N-VinylpyrrolidonCopolymere, hydroxylgruppenhaltiges PAN, Acrylnitril-Vinylacetat-Copolymere, Acrylnitril-Acrylester-Copolymere, Polyallylverbindungen, Polydiallylphthalat, Polytrisallylcyanurat, Poly-$\alpha$-cyanoacrylat, Polydimethylaminoethylmethacrylat und Copolymere mit Acrylnitril, Methylmethacrylat-Laurylmethacrylat-Copolymere, para-Acetaminophenylethoxymethacrylat-Methylmethacrylat-Copolymere, Glykoldimethacrylat-Methacrylat-Copolymere, Poly-2-hydroxyethylmethacrylat, 2-Hydroxyethylmethacrylat-Methylmethacrylat-Copolymere, Glykolmethacrylat-Glykoldimethylmethacrylat-Copolymere, HEMA-Styrol-Block- und Pfropfcopolymere, Poly-N,N'-p,p'-oxydiphenylenmellitimid, Polydiethylenglykolbisallylcarbonat, aliphatische Polyether, Polyoxymethylene, Polyoxyethylen, Polyfluoral, Polychloral, Polyethylenoxid, Polytetrahydrofuran, Polypropylenoxid, Ethylenoxid-Propylenoxid-Copolymere, Propylenoxid-Allylglycidylether-Copolymere, Polyepichlorhydrin, Ethylenoxid-Epichlorhydrin-Copolymere, Poly-1,2-dichlormethyl-ethylenoxid, Poly-2,2-bis-chlormethyl-oxacyclobutan, Epoxid-Harze, Bisphenol-A-diglycidylether, epoxidiertes Phenol-Formaldehyd, Kresol-Formaldehyd-Harze, Vernetzung mit Carbonsäureanhydriden, Aminen wie Diethylentriamin, Isophorondiamid, 4,4'-Diaminodiphenylmethan, aromatische Polyether, Polyphenylenoxide, Polyphenol, Phenoxyharze, aliphatische Polyester, Polylactid, Polyglykolid, Poly-$\beta$-propionsäure, Poly-$\beta$-D-hydroxybutyrat, Polypivolacton, Poly-$\epsilon$-caprolacton, Polyethylenglykoladipat, Polyethylenglykolsebacat, Ungesättigte Polyester aus Maleinsäureanhydrid, Phthalsäureanhydrid, Isophthalsäure, Terephthalsäure oder HET-Säure mit Ethylenglykol, 1,2-Propylenglykol, Neopentylglykol, oxyethylierte Bisphenole oder Cyclododecandiol, Vernetzung ungesättigter Polyesterharze oder Vinylesterharze durch Copolymerisation von ungesättigten Polyestern mit Styrol, Methacrylat, Vinylmonomeren, Vinylacetat, Methylmethacrylat, Polycarbonat aus Bisphenol-A sowie dessen Derivaten und Polyether, Polyester, segmentierte Polycarbonate aus Bisphenol-A sowie dessen Derivaten, und aliphatischen Polyethern sowie aliphatischen Polyestern (siehe oben), Polyethylenglykolterephthalat (PET) oberflächenmodifiziert, mit Acrylsäure gepfropft oder durch partielle Hydrolyse, der Oberfläche von PET, Polybutylenglykolterephthalat, Polyethylenglykolterephthalatadipat, Polyethylenglykolterephthalat segmentiert mit Polyetherblöcken und aliphatischen Polyesterblöcken und Polytetrahydrofuranblöcken, Poly-p-hydroxybenzoat, Hydroxybenzoesäure-Isophthalsäure-Copolymere, Hydroxybenzoesäure-Hydrochinon-Copolymere, Hydroxybenzoesäure-Terephthalsäure-Copolymere, Hydroxybenzoesäure-p,p'-Diphenylether-Copolymere, Polyvinylpyrrolidon, Polyvinylpyrroliden-Maleinsäureanhydrid-Copolymere, Alkydharze aus Glycerin, Trimethylpropan, Pentaerythrit, Sorbit mit Phthalsäure, Bernsteinsäure, Maleinsäure, Fumarsäure, Adipinsäure und Fettsäure aus Leinöl, Ricinusöl, Sojaöl, Kokosöl, aliphatische Polysulfide -(R-Sx-) x = Schwefelgrad, aromatische Polysulfide, Polythio-1,4-phenylen, aromatische Polysulfidether aus Phenol und Thiophen, Polyethersulfone, Polysulfo-1,4-phenylen, Poly-p-phenylensulfon, Polyimine, Polyethylenimin, verzweigte Polyethylenimine, Polyalkylenimine, Polyamide, Polyhexamethylenadipamid, Polyhexamethylensebacamid, Polyhexamethylendodecandiamid, Polytridecanbrassylamid, Versamide aus pflanzlichen Ölen mit Diaminen und Triaminen, Polyamid aus $\omega$-Aminocarbonsäuren mit $\alpha,\beta,\gamma,\delta$-Aminocarbonsäuren oder Lactamen, Terephthalsäure-m-Aminobenzamid-Copolymere, Terephthalsäure-m-Phenylendiamin-Copolymere, Polyamidhydrazide, z.B. aus Isophthalsäure und m-Aminobenzhydrazid, Polypiperazinamide, z.B. aus Fumarsäure und Dimethylpiperazin, Polybenzimidazole aus Terephthalsäure und Tetraaminobenzol (substituiert), oder aus Diaminodiphenylether und Dichlordiphenylsulfon (substituiert und cyclisiert) oder aus m-Phenylenisophthalamid und Terephthalamid, Polyimide, z.B. aus Pyromellitsäuredianhydrid, Methoxy-m-phenylendiamin, Pyrone, z.B. aus Pyromellitsäuredianhydrid und Diaminobenzidin, aromatische Polyamide, Poly-m-phenylenisophthalamid, Poly-p-benzamid, Poly-p-phenylenterephthalamid, m-Aminobenzoesäure-p-Phenylendiamin-Isophthalsäure-Copolymere, Poly-4,4'-diphenylsulfonterephthalamid aus Terephthalsäure und Hexamethylentetramin, Terephthalsäure und Gemischen aus 2,2,4-Trimethylhexamethylendiamin und 2,4,4-Trimethylhexamethylendiamin, aus Terephthalsäure, Diaminomethylennorbonen und $\epsilon$-Caprolactam, aus Isophthalsäure und Laurinlactam, aus Isophthalsäure und Di-4-(cyclohexylamino-3-methyl)-methan, aus 1,12-Decandisäure und 4,4'-Diaminodicyclohexylmethan, aromatische Polyamide mit Heterocyclen, z.B. Dicarbonsäuredichlorid, Terephthalsäure und Isophthalsäure, diaminhaltige Heterocyclen, mit Oxidazol-, Triazol-, Bithiazol- und Benzimidazol-Strukturen, 3-(p-Aminophenyl)-7-amino-2,4-(1H,3H)-chinazolindion + Isophthalsäure, Polyaminosäuren, Poly-methyl-L-glutamat, Poly-L-glutaminsäure u.a., Copolypeptide, z.B. aus Glutaminsäure und Leucin, Glutaminsäure und Phenylalanin, Glutaminsäure und Valin, Glutaminsäure und Alanin, Lysin und Leucin, p-Nitro-D,L-phenylalanin und Leucin u.a., Polyharnstoffe aus Diisocyanaten mit Diaminen und Harnstoffen, Polyurethan aus aliphatischen und aromatischen Diisocyanaten und bifunktionellen und trifunktionellen hydroxylhaltigen aliphatischen Polyestern (siehe oben) und aliphatischen Polyethern (siehe oben), und gegebenenfalls Modifizierung mit bifunktionellen aminogruppenhaltigen, hydroxylgruppenhaltigen und carboxylgruppenhaltigen Substanzen, z.B. Hexamethylendiisocyanat, Diphenylmethandiisocyanat, Toluylendiisocyanat-2,4 und -2,6, Tolidindiisocyanat, Xylylendiisocyanat, Glycerin, Ethylenglykol, Diethylenglykol, Pentaerythrit, 3-Dimethylamino-1,2-propandiol, und Kohlehydrate, aliphatische und aromatische Dicarbonsäuren und deren Derivate, o,m,p-Phenylendiamin, Benzidin, Methylen-bis-o-chloroanilin, p,p'-Diaminodiphenylmethan, 1,2-Diaminopropan, Ethylendiamin, Aminoharze aus Harnstoff und cyclischen Harnstoffen, Melamin, Thioharnstoff, Guanidin, Urethan, Cyanamid, Säureamide und Formaldehyd sowie höhere Aldehyde und Ketone, Silicone, Polydialkylsiloxan, Diarylsiloxan und Alkylarylsiloxan, wie Dimethyl-, Diethyl-, Dipropyl-, Diphenyl-, Phenylmethyl-siloxan, Silicone mit funktionellen Gruppen, z. B. Allylgruppen, $\gamma$-substituierte Fluorsilicone mit Aminogruppen und Vinylgruppen, z.B. Aminopropyltriethoxysiloxan, 2-Carboxylpropylmethylsiloxan, Blockpolymere mit Dimethylsiloxaneinheiten und Polystyrol- oder Polycarbonatblöcken, Dreiblockcopolymere aus Styrol, Butylacrylat mit $\alpha,\omega$-Dihydroxypolydimethylsiloxan, 3,3,3-Trifluorpropylmethylsiloxan, Avocane (90 % Polypropylenglykol und 10 % Siloxan), Blockcopolymere aus Silicon und Polycarbonat, Cellulose und Cellulosederivate, z.B. Celluloseacetat, Perfluorbutyrylethylcellulose, Perfluoracetylcellulose, Perfluoracetylcellulose, Cellulosenitrat, Carboxymethylcellulose, regenerierte Cellulose, regenerierte Cellulose aus Viskose und ähnliche Cellulosederivate, Agarose, Polysaccharide wie Carrageenane, Dextrane, Mannane, Fructosane, Chitin, Pectine, Glykosaminoglykane, Stärke, Glykogen, Alginsäuren, sowie alle Deoxypolysaccharide wie Halogen-deoxypolysaccharide, Amino-deoxypolysaccharide oder Sulfhydryl-deoxypolysaccharide und deren Derivate, Murein, Proteine, z.B. Albumin, Gelatine, Kollagen I - XII, Keratin, Fibrin und Fibrinogen, Casein, Plasmaproteine, Milchproteine, Strukturproteine aus tierischem und pflanzlichen Geweben, Sojaproteine, Proteine aus der Nahrungsmittelindustrie.

Die erweiterte Auswahl von Polymeren ergibt sich dadurch, daß oben aufgeführte Polymere, die aus den verschiedenen Monomerbausteinen synthetisiert werden, mit weiteren bisher bekannten Monomeren copolymerisiert werden (es werden darunter die Monomeren verstanden, die in dem Buch Funktional Monomers, Ed. R.H. Yocum u. E.B. Nyquist, Vol I und II, Marcel Dekker, New York 1974 aufgeführt sind). Des weiteren können die oben aufgeführten Polymeren durch Pfropfung, durch polymeranaloge Reaktionen und durch Herstellung von weiteren Blockcopolymeren und Pfropfcopolymeren partiell oder voll modifiziert werden. Außerdem können Polymermischungen, Legierungen, beschichtete Polymere und Polymere in Form verschiedener Verbundwerkstoffe hergestellt werden. Diese Polymere können durch energiereiche Strahlung, Belichtung, Oxidation, hydrolytischem Abbau, durch photochemische Reaktionen, durch Halogenierung, Sulfochlorierung, Chlormethylierung, durch Umsetzung mit Radikalbildnern, u.ä., Oberflächen-modifiziert werden.

Weiterhin können Polymerderivate mit bi- und polyfunktionellen Verbrückungsreagenzien hergestellt werden, wie sie zur Herstellung von reaktionsfähigen Polymeren nach den Methoden der Peptid-, Protein-, Polysaccharid- und Polymerchemie bekannt sind. Im folgenden ist eine Auswahl der zur Derivatisierung von Polymeren verwendbaren funktionellen Gruppen oder Vernetzermolekülen gegeben:

Phosgen, Formaldehyd, Glyoxal, Acrolein, Glutardialdehyd, Azide, aktivierte Ester, Anhydride, Säurechloride, Ester, gemischte Anhydride, Bromcyan, Difluordinitrobenzol, Thioisocyanat, Epoxid, Imid, Isocyanate, Urethiongruppen, Diisocyanate, Triisocyanate, Maleinimid, Dicyclohexylcarbodiimid, N,N'-Bis(trimethylsilylschwefeldiimid), Peroxide, Vinylketogruppen, aromatische Diazoverbindungen, Vinylsulfon, Trichlortriazin, Monochlortriazin, Dichlortriazin, Bromacrylamid, Difluorchlorpyrimidin, Trichlorpyrimidin, Dichlorchinoxalin, Chloracetylaminogruppen, Chloracetylharnstoff, $\beta$-Halogenpropionamid, $\alpha,\beta$-Dihalogenpropionamid, $\beta$-quaternäres Ammoniumpropionamid, $\beta$-Sulfatopropionamid, $\beta$-Sulfonylpropionamid, substituierte Alkan-dicarboxamide, substituierte Alkan-monocarboxylate, substituierte Cycloalkan-carboxamide, Alken-monocarboxamide, Arylamide, Crotonamide, substituierte Acrylamide, Mono-, Di- und Trihalogen-acrylamide, substituierte Crotonamide, Alken-dicarboxamide, cyclische Halogenmaleinimide, Alkin-carboxamide, substituierte aliphatische Ketone, Amide von substituierten aliphatischen Sulfonsäuren, substituierte Methansulfonamide, substituierte Ethansulfonamide, $\beta$-Sulfatoethylsulfonamide, $\beta$-Thiosulfatoethylsulfonamide, quaternäres Ammoniumethansulfonamid, Vinylsulfonamid, $\beta$-Chlorvinylsulfonamid, Ester von reaktiven aliphatischen Sulfonsäuren, $\beta$-substituierte Ethylsulfone, $\beta$-Thiosulfatoethylsulfone, $\beta$-Halogenvinylsulfon, $\beta$-substituierte Ethylaminderivate, $\beta$-Sulfatoethylamin, $\beta$-Halogenethylpyrazolon, N-($\beta$-Halogenethyl)-amide, N-($\beta$-Sulfatoethyl)-amide, $\beta$-substituierte Ethylammonium-Verbindungen, $\beta$-substituierte Ethylamide von Sulfonsäuren, N,$\beta$-Halogenethylsulfonamide, $\beta,\gamma$-Dihalogenpropionylamide von Sulfonsäuren, $\beta$-Sulfatoethylamide von Sulfonsäuren, Ethylenimin und Ethylenimin-Verbindungen, Allylgruppen, Propargylgruppen, Diallylphthalat, Triallylcyanurat, Benzylderivate, 2-substituierte Thiazolcarbonsäuren, Chlorsulfonylpyridin, 4-Substituiertes-3,5-dicyano-2,6-dichlorpyridin, 2,6-Bis(methylsulfonyl)-pyridin-4-carbonylchlorid, Chlorpyridazin, Dichlorpyridazon, 1-Alkyl-4,5-dichlor-6-pyridazon, Chlor- und Brom-pyrimidin, 3-(2',4',5'-Trichlorpyrimidyl-(6')-amino)-anilin, 4,5,6-Trichlorpyrimidin-2-carbonylchlorid, Trifluorpyrimidin, Trifluorchlorpyrimidin, 2-Chlortriazinyl-Derivate, 2-Chlor-4-alkyl-s-(triazinyl-6-aminocarbonsäure), 2-Chlorobenzothiazolcarbonyl, 6-Amino-2-fluorbenzothiazol, 2-Methylsulfonyl-6-aminobenzothiazol, 2,3-Dichlorchinoxalin-6-carbonylchlorid, 1,4-Dichlorphthalazin-6-carbonylchlorid, 3-Chloro-1,2,4-benzotriazin-1-N-oxid-7-carbonylchlorid, Fluor-2-nitro-4-azidobenzol, Sulfonsäureester, N-Sulfonylharnstoffe, Thiosulfato-S-alkylester, N-Methylolharnstoff, N,N'-Dimethylol-glyoxal-monourein, Terephthaldialdehyd, Mesitylentrialdehyd, Isothiuroniumgruppen, Triacylformal.

Die oben aufgeführten synthetischen Polymere und Biopolymere sowie die daraus hergestellten Polymerderivate mit den oben aufgeführten Vernetzern bzw. funktionellen Gruppen von Vernetzern oder Verfahren zur Oberflächenmodifizierung von Polymeren könen erfindungsgemäß zur Verankerung von HS I eingesetzt werden. Analog kann HS I mit den oben aufgeführten Vernetzermolekülen bzw. den funktionellen Gruppen von Vernetzern und den Verfahren zur Oberflächenmodifizierung zur Modifizierung von HS I für die erfindungsgemäße Verankerung an Polymere eingesetzt werden. Die kovalente Verbrückung von HS I an polymere Substrate kann entweder mit der Querbrückenlänge "O" (Selbstquervernetzung oder Vernetzung nur unter Beteiligung der funktionellen Gruppen des polymeren Substrates und des HS I), oder mit der Querbrückenlänge größer als O unter Verwendung von hydrophilen oder hydrophoben, amphiphilen, geladenen, ungeladenen, flexiblen oder sperrigen Spacern in einer Größenordnung 0,2 - 5 nm erfolgen.

Die Verankerung von HS I an Polymere oder Polymerderivate kann weiterhin erfolgen über

1. salzartige Bindung an positiv-geladene Gruppen von Polymeren, z.B. quaternäre Ammoniumgruppen und Phosphoniumgruppen,

2. Chelatbindung nach den bekannten Methoden zur Herstellung von Chelatbindungen mit funktionellen Gruppen von Makromolekülen und Metallen,

3. über hydrophobe Interaktionen durch Derivatisierung von HS I und/oder Polymeroberflächen mit gesättigten und/oder ungesättigten langkettigen Kohlenwasserstoffen mit einer Kettenlänge von 8 - 30 Methylengruppen, versehen mit den bekannten funktionellen Gruppen, oder mit cyclischen oder heterocyclischen, gesättigten oder ungesättigten Kohlenwasserstoffen mit einer Anzahl von 6 - 50 Kohlenstoffatomen.

Weiterhin kann die Verankerung von HS I über Adsorption, Einschluß in polymere Netzwerke, Einkapselung oder physikalische Vermischung nach den Methoden der Kunststoffverarbeitung erfolgen.

Für den erfindungsgemäßen Einsatz der hämokompatiblen Substrate in Form von Fasern, Hohlfasern, Membranen, Organersatzteilen, Kanülen, Spritzen, Schläuchen, Blutbehältern oder in anderer Form werden die verwendeten oder die hergestellten Polymeren nach ihren für den Verwendungszweck geeigneten physikalischen, mechanisch-technologischen und chemischen Eigenschaften ausgewählt.

Beispiele

Die nachfolgenden Beispiele dienen zur Erläuterung der vorliegenden Erfindung. Dabei wurde so vorgegangen, daß zunächst die Gewinnung von HS I zwecks erfindungsgemäßer Verankerung an polymere Substrate beschrieben wurde. Danach wurde an wenigen ausgewählten Beispielen die kovalente Verankerung an polymere Substrate dargestellt, wobei zum besseren Verständnis überwiegend eine Polymerklasse beschrieben wurde.

Beispiel 1: Gewinnung von HS I aus kultivierten bovinen AortaEndothelzellen zur Verankerung an polymere Substrate

Rinderaortae werden von frisch geschlachteten Tieren entnommen. Die Aortae werden von Fettgewebe etc. befreit und die Aa. intercostales mit Klemmen ligiert. Die Aortae werden dann mit 3 x 50 ml sterilem PBS gespült und an einem Ende mit einer Klemme ligiert. Eine sterile Lösung von 0,1 % bovinem Pankreas-Trypsin (Boehringer Mannheim) in 1 mM EDTA / PBS wird von oben in die Aortae gefüllt und das zweite Ende ligiert. Die Aortae werden dann 7,5 min bei 37°C in einem Wasserbad inkubiert.

Eine der Aortae-Endligaturen wird entfernt und die Flüssigkeit wird mit einer sterilen Pipette entnommen. Die in der Lösung suspendierten Zellen werden bei 800 x g und 37°C 10 min abzentrifugiert, in sterilem Dulbecco's modified essential medium (DMEM) + 10 % fetalem Rinderserum in Gegenwart von 10 000 U Penicillin und 10 000 U Streptomycin (Endothelzellmedium) bei 37°C aufgenommen.

Eine sterile 75 cm$^2$ Gewebekulturflasche wird mit 20 ml Endothelzellmedium 1 h bei 37°C inkubiert. Zu dieser Flasche werden die abzentrifugierten Zellen, suspendiert in 1 ml Endothelzellmedium, gegeben. Die Flasche wird dann in einem Inkubator bei 37°C in einer Atmosphäre von 5 % $CO_2$ und gesättigtem Wasserdampf zur Aufzüchtung der Zellen aufbewahrt.

Die Zellen werden morphologisch und durch Färbung mit antibovinem Faktor VIIIa Antikörpern (Dianova, Hamburg) charakterisiert.

Die Zellen werden zur Konfluenz gezüchtet, mit sterilem PBS gewaschen, von der Unterlage gelöst durch Inkubation mit 2 ml 0,1 % Trypsin in 1 mM EDTA / PBS für 3 min bei 37°C, mit 5 ml Endothelzellmedium versetzt, bei 800 x g abzentrifugiert, in 9 ml Endothelzellmedium resuspendiert und in jeweils 3 ml dieser Suspension in drei neue 75 cm$^2$ Gewebekulturflaschen ausgesät, wie oben beschrieben.

Zur Massenkultur werden die Zellen in Rollerflaschen ausgesät, in der gleichen Weise, wie für die Anzüchtung in den 75 cm$^2$ Flaschen beschrieben. Etwa 10$^9$ Zellen werden für eine HS I-Präparation eingesetzt. Zur Vorbereitung für die HS I-Präparation werden die Zellen mit 1 mM EDTA in PBS von der Unterlage gelöst, durch 3mal 30 s Ultraschallbehandlung bei 0°C mit jeweils 1 min Unterbrechung zwischen jedem Ultraschall homogenisiert, bei 10 000 x g abzentrifugiert und der Überstand auf 2 % SDS eingestellt. Diese Lösung (etwa 5 ml) wird auf eine Sepharose-CL-6B-Säule (5 x 100 cm) gegeben. Eluiert wird mit 0,1 % SDS, 0,1 M Tris/HCl, 1 mM PMSF, 1 mM EDTA pH 7,5. Fraktionen von 20 ml werden aufgefangen bei einer Flußgeschwindigkeit von 100 ml/h.

Der Ausfluß der Säulenchromatographie (Fraktion 35 - 50) wird in 80 % Ethanol präzipitiert, bei 10 000 x g abzentrifugiert, im Wasserstrahlvakuum getrocknet und in 50 ml 7 M Harnstoff, 0,1 % CHAPS (3-((3-Cholamidopropyl)-dimethylammonio)-1-propansulfonat), 0,1 M Tris/HCl, 1 mM PMSF, 1 mM EDTA pH 7,3 gelöst (Lösungspuffer) und auf eine DEAE-Cellulose-Säule (2,5 x 5 cm) gegeben. Die Säule wird mit 100 ml des Lösungspuffers äquilibriert und dann mit einem linearen Gradienten von 0 - 1 M NaCl auf der Basis des Lösungspuffers eluiert (250 + 250 ml). Die Fraktionen, die zwischen 0,45 - 0,6 M NaCl eluieren, werden gesammelt, in 80 % Ethanol präzipitiert, bei 10 000 x g abzentrifugiert und im Vakuum getrocknet.

Die Substanz wird in 12,8 ml 0,1 M Tris/HCl, 4 M Guanidiniumhydrochlorid und Cäsiumchlorid der Dichte 1,4 g/ml gelöst und in einem SW 40 Rotor (Beckmann) bei 35 000 UpM 60 h zentrifugiert. Das Zentrifugationsröhrchen wird am Boden punktiert und Fraktionen von 1 ml werden gesammelt. Die Fraktionen mit einer Dichte von größer als 1,4 g/ml werden gesammelt, in 80 % Ethanol präzipitiert, bei 10 000 x g abzentrifugiert und im Vakuum getrocknet.

Die Substanz wird in 2 ml 0,1 M Tris/HCl 1 mM PMSF, 1 mM EDTA pH 7,5 gelöst und mit 1 U Chondroitinase ABC und 100 U RNAse (Boehringer Mannheim) 1 h bei 37°C inkubiert. Danach wird die Substanz, wie oben beschrieben, an Sepharose-CL-6B rechromatographiert. Die Ausbeute beträgt im Mittel etwa 14 mg/$10^9$ Endothelzellen.

Beispiel 2: Gewinnung von HS I-Polysaccharid aus konditioniertem humanen Umbilical-Endothelzellmedium

Die Umbilicalvenen aus frischen humanen Nabelschnüren werden mit sterilem PBS gespült, und dann mit einer sterilen Lösung von 10 000 U Kollagenase (Boehringer Mannheim) in Cord-Puffer 5 min bei 37°C perfundiert. Die Zellen werden bei 37°C und 800 x g 5 min abzentrifugiert.

Menschliche Seren werden aus dem menschlichen Blut durch Koagulation und Zentrifugation bei 2000 x g gewonnen. Die Seren dürfen in einem SMA-12-Profil keine pathologischen Auffälligkeiten zeigen.

Boviner Wachstumsfaktor wird aus 10 Rinderhypothalami von frisch geschlachteten Tieren durch Homogenisierung und Extraktion mit Aceton/Chloroform 2 : 1 gereinigt. Der in dem Entfettungsmittel unlösliche Rückstand wird in Mengen von 15 - 50 mg/l Kulturmedium eingesetzt.

Menschliches Fibronectin wird aus menschlichem Plasma gewonnen. Dazu wird 10 g Gelatine (Sigma) in 50 ml 0,2 M NaAc bei 65°C gelöst. 200 ml Sepharose CL-2B wird mit 10 g Bromcyan bei einem pH von 10,5 10 min umgesetzt. Das Gel wird danach mit 10 l 0,2 M NaAc gewaschen und mit der Gelatine-Lösung zusammen bei 4°C 24 h geschüttelt. Anschließend wird nach Zugabe von 5 ml Aminoethanol 7 h weitergeschüttelt. Das Gel wird mit 4 M Guanidiniumchlorid und anschließend mit PBS gewaschen (je 1 l). 1 l menschliches Plasma wird mit dem Gel zusammen 24 h bei 4°C geschüttelt. Das Gel wird anschließend mit 5 l PBS auf der Fritte gewaschen und mit 200 ml 1 M NaCl eluiert.

Das Eluat wird gegen 3 x 5 l PBS dialysiert und durch Filtration über einen 0,2 µm-Filter sterilisiert.

Die humanen Endothelzellen werden in 20 ml DMEM + 10 % menschlichem Serum in Gegenwart von 10 000 U Penicillin, 10 000 U Streptomycin und Wachstumsfaktor suspendiert. Eine 75 cm$^2$ Gewebekulturflasche wird mit 2 ml der Fibronectin-Lösung bei Raumtemperatur 3 h inkubiert. Die Lösung wird entfernt, die Flasche mit PBS gewaschen und die Zellen hinzugegeben. Die Flasche wird in einen Inkubationsschrank zur Aufzüchtung der Zellen gegeben (siehe oben).

Die Passagierung und die Massenkultivierung in Rollerflaschen erfolgt mit den gleichen Medien und Kulturgefäßpräparationen, sowie Zellkulturtechniken, wie beschrieben.

Die gesammelten konditionierten Medien (etwa 25 l) werden mit 10 M NaOH auf 0,5 M NaOH eingestellt und 12 h bei 4°C inkubiert. Das Medium wird anschließend mit 10 M HCl auf pH 1,5 bei 4°C eingestellt und bei 10 000 x g abzentrifugiert. Der Überstand wird mit 10 M NaOH neutralisiert und an Amicon PM 10 Membranen ultrafiltriert bis zu einem Volumen von 100 ml. Die Lösung wird auf 80 % Ethanol eingestellt, 12 h bei 4°C stehengelassen und bei 10 000 x g abzentrifugiert. Der Niederschlag wird in PBS aufgenommen, 1 h mit 10 U Chondroitinase ABC bei 56°C inkubiert und auf einer Dowex 1 x 2 Säule (20 ml Gel) gegeben. Die Säule wird mit je 20 ml 1 M NaCl und 4 M NaCl gewaschen. Das 4 M NaCl Eluat wird gegen 3 x 1 l Wasser dialysiert und lyophilisiert. Die Ausbeute beträgt etwa 1,2 mg.

Beispiel 3: Kupplung von HS I an Silicon

1 g Silicon mit freien OH-Gruppen wird mit 18 ml dest. Wasser und 2 ml γ-Aminopropyltriethoxysilan (10 % V/V) versetzt und der pH-Wert mit 6n-HCl zwischen pH 3 und pH 4 eingestellt. Nach der pH-Einstellung wird auf 75°C 2 h erhitzt, gewaschen und getrocknet. 1 g Aminogruppen-haltiges Silicon wird mit 2,5 % wässeriger Lösung von Glutardialdehyd in 0,05 M Natriumphosphat-Puffer versetzt und auf pH 7,0 eingestellt, Reaktionsdauer 60 min. Das aktivierte Silicon wird mit einer 1 % Lösung von HS I unter Rühren 2 - 4 Stunden umgesetzt. Es wird mit 6 M Harnstoff-Lösung gewaschen.

Beispiel 4: Isothiocyanat-Kupplungsreaktion von HS I an Silicon

1 g Aminogruppen-haltiges Silicon wird mit einer 10%igen Lösung von Thiophosgen in Chloroform (V/V) umgesetzt. Die Reaktionsmischung wird mindestens 4 h am Rückfluß gehalten, besser jedoch 12 - 15 h. Es wird mit trockenem Chloroform gewaschen und vakuumgetrocknet. Zum Isothiocyanat-Silicon wird langsam eine wässerige 1 % HS I-Lösung (50-100 mg HS I/g Silicon) gegeben. Der pH-Wert wird auf 8,5 eingestellt und es wird 2 h umgesetzt vor der Wäsche mit einer 6 M Harnstoff-Lösung.

Beispiel 5: Carbodiimid-Kupplung von HS I an Aminogruppenhaltiges Silicon

Zu 1 g Aminogruppen-haltigem Silicon werden 50 ml 0,03 M Phosphorsäure mit einem pH-Wert von 4,0 gegeben. Danach werden 100-200 mg wasserlösliches Carbodiimid und 50-100 mg HS I zur Mischung gegeben und 24 h reagieren gelassen. Es wird, wie oben beschrieben, gewaschen.

Beispiel 6: Triazin-Kupplung von HS I an Aminogruppen-haltiges Silicon

Zu 1 g Aminogruppen-haltigem Silicon werden 10 ml Benzol gegeben, das 0,2 ml Triethylamin und 0,3 g 1,3-Dichlor-5-methoxytriazin enthält. Die Reaktionsmischung wird 2 - 4 h bei 45 bis 55°C gehalten, abdekantiert, mit Benzol gewaschen und getrocknet. Die Kupplung mit HS I (50-100 mg) erfolgt in 0,05 M Phosphat-Puffer bei pH 8 bei 4°C über Nacht. Es wird, wie oben beschrieben, gewaschen.

Beispiel 7: Immobilisierung von HS I über Glutardialdehyd

0,1 - 10 ml verdünnte Glutardialdehyd-Lösungen (0,1 - 5 %) werden zu 1 g dispergierten oder imprägnierten Aminogruppen-haltigem Polymeren und 10 mg HS I gegeben und bei pH 7,4 0,5 - 2 min umgesetzt. Danach wird sofort mit kaltem Wasser gewaschen.

Beispiel 8: Thermische und photochemische Immobilisierung von HS I an Polymere

1 g Fluor-2-nitro-4-azidobenzol wird mit 10 g HS I im Dunkeln bei 50°C und maximal pH 10,5 in 0,05 M Natriumborat-Puffer 16 - 64 h umgesetzt. Das Reaktionsprodukt wird abfiltriert und mit 95 % Ethanol gewaschen. Die Photoimmobilisierung an Polymere erfolgt in einigen Stunden bei 200 - 300 Watt mit einer Quecksilberdampflampe. Danach wird, wie oben beschrieben, gewaschen.

Beispiel 9: Verankerung von HS I über Oxirangruppen an Hydroxyl- und Aminogruppen-haltige Polymere

10 g gewaschenes 2%iges Agarose-Gel wird in 5 ml 2,5 M NaOH-Lösung suspendiert. 20 mg Natriumborhydrid wird zugefügt und danach werden tropfenweise 1,4-Butandioldiglycidylether unter heftigem Rühren zugegeben. Die Reaktion dauert 6 h bei Raumtemperatur. Dann werden 50 ml Aceton und schließlich Wasser zugesetzt. Das Oxiran-Gel wird in 25 ml 0,5 M Natriumbicarbonat suspendiert und 50 mg HS I zugefügt. Die Reaktion dauert 5 h. Das Produkt wird, wie oben beschrieben, gewaschen.

Beispiel 10: Azokupplung von HS I über Arylaminruppen an Polymere

1 g Aminogruppen-haltiges Silicon wird in 25 - 50 ml Chloroform gelöst, das 5 % Triethylamin (V/V) und 1 g p-Nitrobenzoylchlorid enthält. Die Reaktionsmischung wird wenigstens 4 h unter Rückfluß gehalten. Es wird mit trockenem Chloroform gewaschen und unter Rückfluß in einer 5 % Natriumdithionit-Lösung gekocht. Die Azokupplung erfolgt mit 1 g Arylamin-Silicon durch Zugabe von 20 ml 2n-HCl im Eisbad. Es werden 100 mg festes Natriumnitrit zugegeben und 30 min diazotiert. Danach wird mit Eiswasser gewaschen. 100 mg HS I werden bei pH 8,5 in 0,05 M Natriumphosphat gelöst und 1 g diazotiertes Polymeres zugegeben. Es wird zwischen 2 - 18 h gekuppelt. Das Produkt wird, wie oben beschrieben, gewaschen.

Beispiel 1: Herstellung von Polymer-gebundenem HS I durch Copolymerisation mit Alkylierungs- und Acylierungsmonomeren

3,4-Epoxybuten, Acrylsäure-2,3-epoxypropylester, Acrylsäure-2,3-thioglycidylester, 1-Allyloxy-3-(N-ethylenimin)-2-propanol, Acrylsäure-O-succinimidester oder Acrylsäurechlorid, Maleinsäureanhydrid, Chlormaleinsäureanhydrid, Maleinsäureazid oder 3-Brompropen werden in äquimolaren Mengen mit HS I umgesetzt und anschließend mit den üblichen Methoden polymerisiert oder copolymerisiert.

**Patentansprüche**

1. Verfahren zur Herstellung von nicht-thrombogenen Substanzen für die Herstellung von Membranen, Organersatzteilen, Kanülen, Spritzen, Schläuchen, Röhren, Blutbehältern und ähnlichen, für den Einsatz von in der Medizin angewandten Blutbehältnissen durch Verankerung von HS I an Polymere und/oder auf Oberflächen von allen bis heute bekannten Biopolymeren und synthetischen Polymeren, einschließlich deren bis heute bekannten Copolymeren und deren Derivate und reaktiven Derivate,
   **dadurch gekennzeichnet,** daß es sich bei HS I um ein spezifisches Endothelzelloberflächen-Proteopolysaccharid handelt, welches nur von diesen Zellen produziert wird, das drei bis vier Polysaccharidseitenketten, die ein Molekulargewicht von je 35 000 aufweisen und ein zentrales Coreprotein mit einem Molekulargewicht von etwa 55 000 trägt, wobei sich der Polysaccharidanteil dieser Substanz von allen bisher bekannten Strukturpolysacchariden und Glykosaminoglykanen, speziell von allen bekannten Heparinen und Heparansulfaten dadurch unterscheidet, daß er keinerlei Wechselwirkungen mit den Faktoren der Blutgerinnung und keinerlei sonstige biologische Aktivität, die bisher bei Glykosaminoglykanen und speziell Heparinen und Heparansulfaten beobachtet wurde, aufweist.

2. Verfahren nach Anspruch 1, **dadurch gekennzeichnet,** daß etwa 0,01 - 100 μg HS I pro Quadratzentimeter Polymeroberfläche kovalent gebunden werden und zwischen 1 und 80 kovalenten Verankerungsstellen oder Querbrücken zwischen HS I und Polymerem vorliegen.

3. Verfahren nach Anspruch 1, **dadurch gekennzeichnet,** daß die Verankerung von HS I in oder auf dem polymeren Substrat durch Salzbrücken, hydrophobe Interaktionen, Wasserstoffbrückenbindung und van der Waals-Kräfte, Adsorption, Mikroverkapselung oder Netzwerkeinschluß in einer Konzentration von 1 μg - 1000 μg pro Quadratzentimeter stattfindet.

4. Verfahren nach Anspruch 1 - 3, **dadurch gekennzeichnet,** daß HS I an Polymersubstrate unter Ausnutzung der Proteinleisten von HS I verankert wird, wobei bei in-vivo Applikation von Spezies-differentem Material die Länge und Struktur der Proteinleiste des HS I so geschaffen sein muß, daß keine immunologischen oder sonstigen Unverträglichkeitsreaktionen auftreten können.

5. Verfahren nach Anspruch 1 - 3, **dadurch gekennzeichnet,** daß die HS I-Polysaccharidkette an das polymere Substrat kovalent gebunden wird.

6. Verfahren nach Anspruch 1, 2, 4 und 5, **dadurch gekennzeichnet,** daß alle bekannten Verbrückungsreagenzien oder Quervernetzungsmethoden zur Herstellung kovalenter Bindungen zur Verankerung von HS I an Polymeroberflächen eingesetzt werden können, die zur Verankerung von Farbstoffen an Polymere oder Proteinen an Polymere oder Proteinen und Kohlenhydrate an Polymere oder Biopolymere untereinander eingesetzt werden.

7. Verfahren nach Anspruch 1, 2, 4 - 6, **dadurch gekennzeichnet,** daß HS I und/oder das polymere Substrat mit den bekannten proteinchemischen Methoden und den Methoden der Enzymimmobilisierung sowie den Methoden der Polysaccharidchemie und der Polymerchemie aktiviert und anschließend HS I mit dem Polymersubstrat kovalent verbunden wird.

8. Verfahren nach Anspruch 1, 2, 4 - 7, **dadurch gekennzeichnet,** daß die kovalente Verbrückung von HS I an polymere Substrate entweder mit der Querbrückenlänge "O" (Selbstquervernetzung oder Vernetzung nur unter Be teiligung der funktionellen Gruppen des polymeren Substrates und des HS I) oder mit der Querbrückenlänge von größer als O unter Verwendung von hydrophilen oder hydrophoben, amphiphilen, geladenen oder ungeladenen Spacern in einer Größenordnung von 0,2 - 5 nm erfolgt.

9. Verfahren nach Anspruch 1, 3 - 5, **dadurch gekennzeichnet,** daß die salzartige Bindung von HS I an das polymere Substrat entweder direkt über die geladenen funktionellen Gruppen erfolgt oder unter Zuhilfenahme von zwischen HS I und polymerem Subtrat gelegenen, geladenen Substanzen, wie z.B. zwitterionische Substanzen und Polymere, Proteine etc., in einer Größenordnung von 0,15 - 200 nm.

**10.** Verfahren nach Anspruch 1, 3 - 5 und 9, **dadurch gekennzeichnet,** daß die geladene Gruppe entweder direkt am Backbone des polymeren Substrates oder des HS I oder unter Verlängerung des Abstandes zwischen geladener Gruppe und polymerem Substrat bzw. HS I durch Verlängerung der Seitenketten des Polymeren in einer Größenordnung von 0,15 - 500 nm gebunden ist.

**11.** Verfahren nach Anspruch 1, 3 - 5, **dadurch gekennzeichnet,** daß das HS I zunächst an positiv geladene Gruppen bindet, die Bestandteil von Substanzen mit hydrophoben Resten sind, über die eine hydrophobe Bindung an das polymere Substrat zustande kommt.

**12.** Verfahren nach Anspruch 1 - 5, **dadurch gekennzeichnet,** daß HS I an polymere Substrate über salzartige Bindung, Wasserstoffbrükkenbindung, hydrophobe Interaktionen und van der Waals-Kräfte adsorbiert wird und danach die Quervernetzung mit Reagenzien und Methoden nach Anspruch 6 und 8 erfolgt.

**13.** Verfahren nach Anspruch 1 und 3, **dadurch gekennzeichnet,** daß HS I durch Vorpolymerisierung des zu erstellenden Polymersubstrates, welches noch löslich ist, immobilisiert wird, anschließend HS I mit oder ohne Vernetzungsmittel zugegeben und zu einem unlöslichen Polymersubstrat zu Ende polymerisiert wird.

**14.** Verfahren nach Anspruch 1 und 3, **dadurch gekennzeichnet,** daß das HS I im Polymersubstrat nach der Methode der Grenzschichtenpolymerisation, der Flüssigkeitentrocknungsmethode, der Koazervationsmethode, der Phasenmethode und der Liposomentechnik mikroverkapselt wird.

**15.** Verfahren nach Anspruch 1 und 2, **dadurch gekennzeichnet,** daß HS I mit reaktionsfähigen Seitenketten von Vinylmonomeren umgesetzt und anschließend nach den üblichen Methoden mit anderen Monomeren copolymerisiert wird.

**Claims**

**1.** A process to produce non-thrombogenic substances for the production of membranes, spare parts of organs, canules, syringes, pipes, tubes, blood receptacles and the like, for the application of blood containers used in medicine by affixing of HS I to polymers and/or on surfaces of all biopolymers so far known and to synthetic polymers, including copolymers and derivatives and reactive derivatives thereof so far known, characterized in that HS I is a specific proteopolysaccharide on surfaces of endothelium cells and being exclusively produced by said cells, the proteopolysaccharide carrying three to four polysaccharide side chains each having a molecular weight of 35 000 dalton and a central coreprotein of a molecular weight of about 55 000 dalton, the polysaccharide portion of said substance differing from all structural polysaccharides and glycosaminoglycanes so far known, especially from all known heparins and heparansulfates by the fact, that it interacts by no means with the factors of blood coagulation and that it shows no whatsoever other biological activity being discovered hitherto with glycosaminoglycanes and especially with heparins and heparansulfates.

**2.** A process according to claim 1, characterized in that, per $cm^2$ of polymer surface, 0.01 - 100 $\mu$g HS I are covalently linked and in that between 1 and 80 covalent anchoring sites or cross bridges are present between HS I and polymer.

**3.** A process according to claim 1, characterized in that the affixing of HS I in or on the polymer substrate is performed by salt bridges, hydrophobic interactions, hydrogen bonds and Van der Waals forces, adsorption, microencapsulation or network inclusion in a concentration of 1 $\mu$g - 1 000 $\mu$g per $cm^2$.

**4.** A process according to claims 1-3, characterized in that HS I is affixed to polymer substrates by using the protein ties of HS I, and, when material of different species is applied invivo, the length and structure of the said protein tie of HS I is to be developed thus that immunological and other reactions of incompatability cannot occur.

**5.** A process according to claims 1-3, characterized in that the polysaccharide chain of HS I is covalently linked to the polymer substrate.

**6.** A process according to claims 1, 2 and 5, characterized in that, in order to affix HS I to polymer surfaces, all known bridging reagents or crossbridging methods can be used that are employed to affix dyes to polymers, or proteins to polymers, or proteins and carbohydrates to polymers, or biopolymers one to another.

7. A process according to claims 1, 2 and 4-6, characterized in that HS I and/or the polymer substrate is activated by using all known methods of protein chemistry and the methods of immobilization of enzyms as well as the methods of polysaccharide chemistry and polymer chemistry and in that successively HS I is covalently linked to the polymer substrate.

8. A process according to claims 1, 2 and 4-7, characterized in that the covalent bridging of HS I to a polymer substrate is performed by the length of the cross bridge "O" (self-crosslinking or linking only by using the functional groups of the polymer substrate and HS I) or by the length of the cross bridge >0 by using hydrophilic or hydrophobic, amphiphilic, loaded or unloaded spacers in the range of 0.2 - 5 nm.

9. A process according to claims 1 and 3-5, characterized in that the saltlike bond of HS I to the polymer substrate is performed either directly by the loaded functional groups or by the aid of loaded substances situated between HS I and the polymeric substrate, as e.g. zwitterionic substances and polymers, proteins etc. in a range of 0.15 - 200 nm.

10. A process according to claims 1, 3-5 and 9, characterized in that the loaded group is bound either directly to the backbone of the polymeric substrate or to HS I or in elongating the distance between the loaded group and the polymeric substrate or HS I respectively by elongating the side chains of the polymer in a range of 0.15 - 500 nm.

11. A process according to claims 1 and 3-5, characterized in that HS I is firstly binding to positively loaded groups being a part of substances with hydrophobic residues, by which a hydrophobic bond to the polymeric substrate is performed.

12. A process according to claims 1-5, characterized in that HS I adsorbs to polymeric substrates by a saltlike bond, hydrogen bond, hydrophobic interaction and Van der Waals force, and subsequently, the cross linking is performed with reagents and methods according to claims 6 and 8.

13. A process according to claims 1 and 3, characterized in that HS I is immobilized by prepolymerization of the polymeric substrates to be made and which is still soluble and HS I is successively added with or without a crosslinking agent and is completely polymerized to an insoluble polymeric substrate.

14. A process according to claims 1 and 3, characterized in that HS I is encapsulated in the polymeric substrate according to the method of interphase polymerization, the liquid drying method, the coacervation method, the phase method and the liposome technique.

15. A method according to claims 1 and 2, characterized in that HS I is reacted with the reactive side chains of vinylmonomers and is successively copolymerized with other monomers according to customary methods.

**Revendications**

1. Procédé de fabrication de substances non thrombogènes pour la fabrication de membranes, d'éléments de prothèses d'organes, de canules, de seringues, de tuyaux souples, de tubes, de récipients à sang et analogues, pour l'utilisation de contenants à sang utilisés en médecine, par ancrage de HS I à des polymères et/ou sur des surfaces de tous les biopolymères et Polymères synthétiques connus jusqu'à présent, y compris leurs copolymères connus jusqu'à présent et leurs dérivés et dérivés réactifs, caractérisé en ce que le HS I est un protéopolysaccharide spécifique superficiel des cellules endothéliales qui n'est produit que par ces cellules, qui porte trois à quatre chaînes latérales de polysaccharide qui ont chacune un poids moléculaire de 35 000 et une protéine d'âme d'un poids moléculaire d'environ 55 000, la partie polysaccharide de cette substance se distinguant de tous les polysaccharides de structure et glycosaminoglycanes connus jusqu'à présent, spécialement de toutes les héparines et tous les héparanesulfates connus, en ce qu'elle ne présente aucune interaction avec les facteurs de la coagulation sanguine et aucune autre activité biologique qui a été observée jusqu'à présent dans le cas des glycosaminoglycanes et spécialement des héparines et héparanesulfates.

2. Procédé suivant la revendication 1, caractérisé en ce qu'environ 0,01 à 100 $\mu$g de HS I sont liés par covalence par $cm^2$ de surface de polymère et il existe entre 1 et 80 points d'ancrage covalents ou réticulations entre le HS I et le polymère.

3. Procédé suivant la revendication 1, caractérisé en ce que l'ancrage du HS I dans ou sur le substrat polymère a lieu par des ponts de sel, par des interactions hydrophobes, par formation de ponts hydrogène et forces de van der Waals, par adsorption, par microencapsulation ou par insertion dans le réseau en une concentration de 1 μg à 1000 μg par cm².

4. Procédé suivant l'une quelconque des revendications 1 à 3, caractérisé en ce que le HS I est ancré aux substrats de polymère par recours aux brins de protéine du HS I, la longueur et la structure du brin de protéine du HS I devant être telles qu'aucune réaction d'incompatiblité immunologique ou autre ne puisse se produire lors de l'application in vivo d'un matériau différent de l'espèce.

5. Procédé suivant l'une quelconque des revendications 1 à 3, caractérisé en ce que la chaîne de polysaccharide du HSI est liée par covalence au substrat polymère.

6. Procédé suivant l'une quelconque des revendications 1, 2, 4 et 5, caractérisé en ce que tous les réactifs de pontage ou procédés de réticulation connus pour la formation de liaisons covalentes, qui sont appliqués à l'ancrage de colorants à des polymères, ou de protéines à des polymères, ou de protéines et d'hydrates de carbone à des polymères ou de biopolymères entre eux, peuvent être utilisés pour l'ancrage du HS I aux surfaces des polymères.

7. Procédé suivant l'une quelconque des revendications 1, 2 et 4 à 6, caractérisé en ce que le HS I et/ou le substrat polymère sont activés suivant les procédés connus de la chimie des protéines et les procédés d'immobilisation des enzymes, de même que les procédés de la chimie des polysaccharides et de la chimie des polymères, et le HS I est ensuite lié par covalence au substrat de polymère.

8. Procédé suivant l'une quelconque des revendications 1, 2 et 4 à 7, caractérisé en ce que le pontage covalent du HS I avec les substrats polymères a lieu soit avec la longueur de réticulation "0" (autoréticulation ou réticulation avec la seule participation des radicaux fonctionnels du substrat polymère et du HS I), soit avec une longueur de réticulation supérieure à 0 par l'utilisation d'éléments d'écartement hydrophiles ou hydrophobes, amphiphiles, chargés ou non chargés, dans un ordre de grandeur de 0,2 à 5 nm.

9. Procédé suivant l'une quelconque des revendications 1 et 3 à 5, caractérisé en ce que la liaison saline du HS I au substrat polymère a lieu soit directement par les radicaux fonctionnels chargés, soit par l'intermédiaire de substances chargées, comme des substances et polymères zwitterioniques des protéines etc., disposées entre le HS I et le substrat polymère, dans un ordre de grandeur de 0,15 à 200 nm.

10. Procédé suivant l'une quelconque des revendications 1, 3 à 5 et 9, caractérisé en ce que le radical chargé est lié soit directement au squelette du substrat polymère ou du HS I, soit avec augmentation de la distance entre le radical chargé et le substrat polymère ou le HS I par allongement des chaînes latérales du polymère dans un ordre de grandeur de 0,15 à 500 nm.

11. Procédé suivant l'une quelconque des revendications 1 et 3 à 5, caractérisé en ce que le HS I se lie d'abord à des radicaux à charge positive, qui sont constitutifs de substances à restes hydrophobes, par lesquels une liaison hydrophobe au substrat polymère se forme.

12. Procédé suivant l'une quelconque des revendications 1 à 5, caractérisé en ce que le HS I est adsorbé sur les substrats polymères par liaison saline, par formation de ponts hydrogène, par des interactions hydrophobes et par des forces de van der Waals et la réticulation est réalisée ensuite avec les réactifs et procédés suivant les revendications 6 et 8.

13. Procédé suivant l'une quelconque des revendications 1 et 3, caractérisé en ce que du HS I est immobilisé par prépolymérisation du substrat de polymère à préparer, qui est encore soluble, après quoi du HS I est ajouté avec ou sans agent de réticulation et est polymérisé complètement en un substrat de polymère insoluble.

14. Procédé suivant l'une quelconque des revendications 1 et 3, caractérisé en ce que le HS I est microencapsulé dans le substrat de polymère suivant le procédé de la polymérisation interfaciale, le procédé de la dessiccation des liquides, le procédé de la coacervation, le procédé des phases et la technique des liposomes;

15. Procédé suivant l'une quelconque des revendications 1 et 2, caractérisé en ce que le HS I est mis à réagir avec des chaînes latérales réactives de monomères vinyliques et est ensuite copolymérisé avec d'autres monomères suivant les procédés habituels.